# EUROPEAN PATENT APPLICATION

(11) **EP 4 813 313 A1**
(43) Date of publication of application: **30.09.2026**
(21) Application number: 26165610.2
(22) Date of filing: 17.03.2026
(51) Int. Cl.: A61B 6/42, A61B 6/00, A61B 6/50, A61B 6/04

(54) **INFORMATION PROCESSING APPARATUS, INFORMATION PROCESSING METHOD, AND PROGRAM**

(30) Priority: 28.03.2025 JP 2025057159
(71) Applicant: FUJIFILM Corporation, Tokyo 106-8620 (JP)
(72) Inventor: FUKUDA, Wataru, Kanagawa (JP)
(74) Representative: Dehns Germany Partnerschaft mbB

(57) **Abstract**

Provided are an information processing apparatus (12), an information processing method, and a program that enable high-accuracy removal of an artifact from an energy difference image.

An information processing apparatus according to the present disclosure includes a processor (40), in which the processor is configured to acquire a low-energy image captured by irradiating a subject (M) into which a contrast agent has been injected with radiation having a first energy, and a high-energy image captured by irradiating the subject with radiation having a second energy higher than the first energy, generate an initial energy difference image by performing difference processing on the low-energy image and the high-energy image, extract an artifact component (N1-N3) from the initial energy difference image; and generate an energy difference image by performing the difference processing again based on the artifact component.

## Description

### BACKGROUND OF THE INVENTION

### 1. Field of the Invention

The present disclosure relates to an information processing apparatus, an information processing method, and a program.

### 2. Description of the Related Art

Contrast-enhanced mammography is known in which a low-energy image and a high-energy image are acquired by performing imaging by irradiating a breast into which a contrast agent has been injected with radiation having different energies, and an energy difference image in which a lesion or the like is emphasized by the contrast agent is generated by taking a difference between the two images.

In the energy difference image, an artifact including a non-uniformity due to an influence of a change in thickness of the breast or radiation scattering occurs, which causes a problem of erroneous diagnosis, overlooking of a lesion, and the like (for example, see US8165379B and JP7221981B). US8165379B proposes a technique of analyzing the energy difference image to extract the non-uniformity as a low-frequency component, and effectively removing the non-uniformity by superimposing a correction image of an inverse pattern thereof to emphasize a lesion portion. JP7221981B proposes a technique of acquiring the low-energy image and the high-energy image, and removing the non-uniformity due to an oblique incidence component or a scattered ray component by performing correction according to a length of a transmission path of the radiation.

### SUMMARY OF THE INVENTION

In US8165379B, the energy difference image is analyzed to correct the non-uniformity. Since the cause of the non-uniformity is a physical factor such as scattered rays or a change in thickness, the composite factors cannot be separated in the energy difference image, and thus there is a limit to the estimation accuracy.

In JP7221981B, the non-uniformity is corrected in the low-energy image and the high-energy image before the energy difference image is generated. However, in this method, in order to accurately correct the non-uniformity, accurate information on the oblique incidence component and the scattered ray component is required. In the estimation of these components, physical information such as a shape and a thickness of the subject is essential, but it may be difficult to acquire these pieces of information. In particular, in biopsy imaging, since an opening for passing a biopsy needle is provided in a compression plate for compressing the breast, it is difficult to accurately ascertain the thickness due to an influence of the compression plate. Further, in the biopsy imaging, a grid for removing scattered rays is not used in many cases, and thus a large amount of scattered rays are generated, which makes it more difficult to accurately estimate the scattered ray component.

An object of the technology of the present disclosure is to provide an information processing apparatus, an information processing method, and a program that enable high-accuracy removal of an artifact from an energy difference image.

In order to achieve the above object, an information processing apparatus according to an aspect of the present invention comprises a processor, in which the processor is configured to acquire a low-energy image captured by irradiating a subject into which a contrast agent has been injected with radiation having a first energy, and a high-energy image captured by irradiating the subject with radiation having a second energy higher than the first energy, generate an initial energy difference image by performing difference processing on the low-energy image and the high-energy image, extract an artifact component from the initial energy difference image, and generate an energy difference image by performing the difference processing again based on the artifact component.

The processor may generate the energy difference image by correcting the low-energy image and the high-energy image based on the artifact component and then performing the difference processing.

The processor may extract, as the artifact component, a remaining component obtained by performing, on the initial energy difference image, signal component removal processing of removing a signal component corresponding to the contrast agent.

The processor may perform the signal component removal processing using a high-frequency cut filter.

The initial energy difference image includes a first initial energy difference image and a second initial energy difference image, and the processor may generate the first initial energy difference image by multiplying at least one of the low-energy image or the high-energy image by a weight coefficient and performing the difference processing, and generate the second initial energy difference image by performing the difference processing again using the weight coefficient corrected based on a remaining component obtained by performing, on the first initial energy difference image, signal component removal processing of removing a signal component corresponding to the contrast agent and scattered ray component removal processing of removing a scattered ray component.

The processor may extract, as the artifact component, a remaining component obtained by performing the signal component removal processing on the second initial energy difference image.

The processor may generate the energy difference image by correcting the low-energy image and the high-energy image based on the artifact component and then performing the difference processing.

The processor may generate the energy difference image by performing the difference processing using the corrected weight coefficient.

The processor may correct the weight coefficient based on a thickness component of the subject included in the first initial energy difference image.

The thickness component may be a remaining component obtained by performing the signal component removal processing and the scattered ray component removal processing on the first initial energy difference image.

The processor may perform the signal component removal processing using a high-frequency cut filter.

The subject may be a breast.

An information processing method according to another aspect of the present invention includes acquiring a low-energy image captured by irradiating a subject into which a contrast agent has been injected with radiation having a first energy, and a high-energy image captured by irradiating the subject with radiation having a second energy higher than the first energy, generating an initial energy difference image by performing difference processing on the low-energy image and the high-energy image, extracting an artifact component from the initial energy difference image, and generating an energy difference image by performing the difference processing again based on the artifact component.

A program according to another aspect of the present invention causes a computer to execute a process including acquiring a low-energy image captured by irradiating a subject into which a contrast agent has been injected with radiation having a first energy, and a high-energy image captured by irradiating the subject with radiation having a second energy higher than the first energy, generating an initial energy difference image by performing difference processing on the low-energy image and the high-energy image, extracting an artifact component from the initial energy difference image, and generating an energy difference image by performing the difference processing again based on the artifact component.

According to the technology of the present disclosure, it is possible to provide an information processing apparatus, an information processing method, and a program that enable high-accuracy removal of an artifact from an energy difference image.

### BRIEF DESCRIPTION OF THE DRAWINGS

FIG. 1 is a diagram showing an example of an overall configuration of a radiation image capturing system.
FIG. 2 is a schematic diagram showing a state at a reference position as viewed from the front of a mammography apparatus.
FIG. 3 is a schematic diagram showing an example of a state in which an arm portion of the mammography apparatus is rotated.
FIG. 4 is a diagram showing an example in which an opening is provided in a compression plate for biopsy imaging.
FIG. 5 is an explanatory diagram for describing a concept of contrast enhancement by energy difference processing.
FIG. 6 is a diagram showing an example of an artifact component and a signal component included in an energy difference image in a case of performing biopsy imaging.
FIG. 7 is a block diagram showing an example of a configuration of an information processing apparatus.
FIG. 8 is a block diagram showing an example of functions realized by a controller of the information processing apparatus.
FIG. 9 is a flowchart for describing in detail processing of generating an energy difference image by an analysis unit, an image correction unit, and a difference processing unit.

### DESCRIPTION OF THE PREFERRED EMBODIMENTS

Hereinafter, embodiments of the present disclosure will be described in detail with reference to the accompanying drawings.

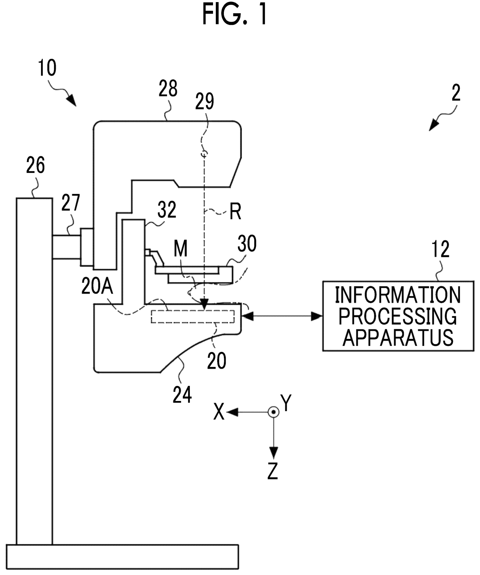

FIG. 1 shows an example of an overall configuration of a radiation image capturing system 2 according to the present embodiment. The radiation image capturing system 2 comprises a mammography apparatus 10 and an information processing apparatus 12. The information processing apparatus 12 is connected to a radiology information system (RIS), a picture archiving and communication system (PACS), and the like (none of which is shown) via a network or the like.

FIG. 1 shows an example of an appearance of the mammography apparatus 10. It should be noted that FIG. 1 shows an example of the appearance in a case in which the mammography apparatus 10 is seen from the left side of a person under an examination.

The mammography apparatus 10 is a radiography apparatus that operates under the control of the information processing apparatus 12 and that irradiates a breast M of the person under the examination, as a subject, with radiation R (for example, X-rays) from a radiation source 29 to capture a radiation image of the breast M.

As shown in FIG. 1, the mammography apparatus 10 comprises an imaging table 24, a base 26, an arm portion 28, and a compression unit 32. A radiation detector 20 is disposed inside the imaging table 24. As shown in FIG. 1, in the mammography apparatus 10, during imaging, the breast M of the person under the examination is positioned on the imaging table 24 by a user such as a radiologic technologist.

The radiation detector 20 detects the radiation R passing through the breast M, which is a subject. Specifically, the radiation detector 20 detects the radiation R passing through the breast M of the person under the examination, entering the imaging table 24, and reaching a detection surface 20A of the radiation detector 20, and generates a radiation image based on the detected radiation R. The radiation detector 20 outputs image data representing the generated radiation image. Hereinafter, the series of operations of irradiating with the radiation R from the radiation source 29 and generating a radiation image via the radiation detector 20 may be referred to as "imaging". The radiation detector 20 may be an indirect conversion type radiation detector that converts the radiation R into light beams and converts the converted light beams into charges, or may be a direct conversion type radiation detector that directly converts the radiation R into charges.

Hereinafter, two directions orthogonal to each other and parallel to the detection surface 20A will be referred to as an X direction and a Y direction. In addition, a direction orthogonal to the X direction and the Y direction will be referred to as a Z direction.

A compression plate 30 that is used for compressing the breast M in a case of performing the imaging is attached to the compression unit 32. The compression plate 30 is moved in a direction approaching or in a direction spaced away from the imaging table 24 by a compression plate drive unit (not shown) provided in the compression unit 32. The compression plate 30 is moved in a direction approaching the imaging table 24 to compress the breast M with the imaging table 24.

The arm portion 28 can be rotated with respect to the base 26 by a shaft portion 27. The shaft portion 27 is fixed to the base 26, and the shaft portion 27 and the arm portion 28 are rotated integrally. Gears are provided in each of the shaft portion 27 and the compression unit 32 of the imaging table 24, and an engagement state between the gears is switched between an engaged state and a disengaged state, so that a state in which the compression unit 32 of the imaging table 24 and the shaft portion 27 are connected to each other and are rotated integrally and a state in which the shaft portion 27 is separated from the imaging table 24 and idles can be switched. It should be noted that the elements for switching between transmission and non-transmission of power of the shaft portion 27 are not limited to the gears, and various mechanical elements can be used. The arm portion 28 and the imaging table 24 can be separately rotated relative to the base 26 with the shaft portion 27 as a rotation axis.

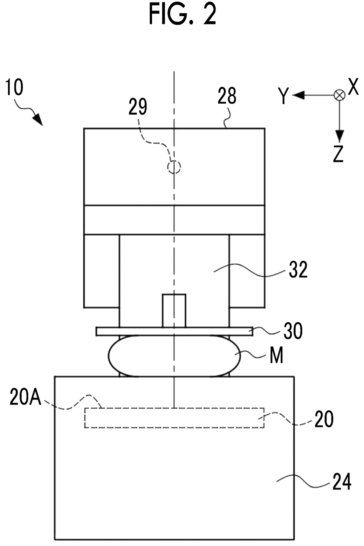

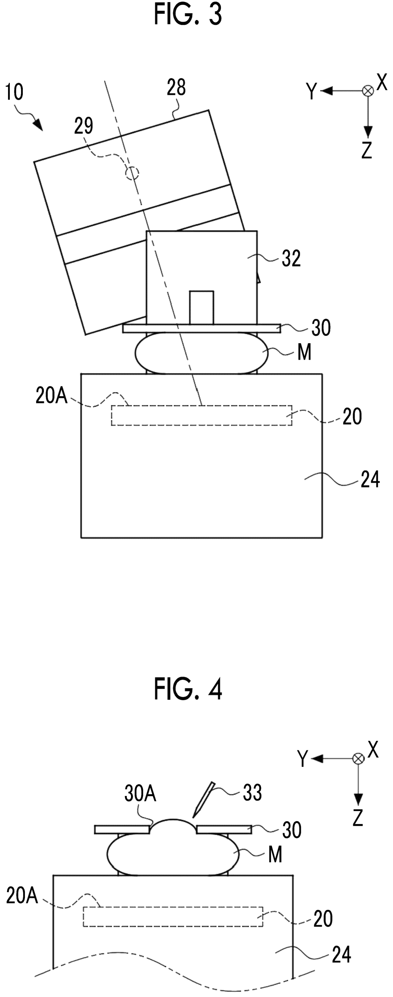

FIGS. 2 and 3 are schematic diagrams of the mammography apparatus 10 as viewed from the front. FIG. 2 shows a state in which the arm portion 28 is not rotated, that is, a reference position, and shows a standard disposition in a case of imaging. On the other hand, FIG. 3 shows an example of a state in which the arm portion 28 is rotated.

The mammography apparatus 10 can perform the imaging on each of the left and right breasts M from a plurality of directions by rotating the arm portion 28. For example, it is possible to perform cranio-caudal (CC) imaging and medio-lateral oblique (MLO) imaging.

FIG. 4 shows an example in which an opening 30A is provided in the compression plate 30 for performing biopsy imaging. The biopsy imaging is an imaging method performed in a biopsy technique for collecting a tissue such as a tumor in the breast M.

In the biopsy imaging, the compression plate 30 is used in order to accurately specify a target site in the breast M and insert a biopsy needle 33 into the site. The compression plate 30 is provided with the opening 30A for passing the biopsy needle 33, and the biopsy needle 33 is inserted into the target site in the breast M through the opening 30A.

Further, in the biopsy imaging, a biopsy needle unit (not shown) for appropriately installing the biopsy needle 33 is attached to the mammography apparatus 10. The biopsy needle unit comprises a mechanism that accurately guides the biopsy needle 33 while performing position adjustment with respect to the opening 30A.

The radiation image capturing system 2 can perform "contrast-enhanced imaging" in which the imaging is performed in a state in which a contrast agent is injected into the breast M. Specifically, the radiation image capturing system 2 has a contrast enhanced digital mammography (CEDM) function of performing contrast enhancement via energy subtraction.

In the contrast-enhanced imaging, a low-energy image (hereinafter, referred to as an "LE image") and a high-energy image (referred to as an "HE image") are acquired by irradiating the breast M into which the contrast agent has been injected with the radiation R having different energies and performing imaging. In the present disclosure, a radiation image captured by the radiation R having the first energy is referred to as an LE image, and a radiation image captured by the radiation R having the second energy higher than the first energy is referred to as an HE image. Hereinafter, in a case where the LE image and the HE image are not distinguished from each other, these images are simply referred to as radiation images.

In the contrast-enhanced imaging, for example, an iodine contrast agent having a K absorption edge of 32 keV is used as the contrast agent. The iodine contrast agent has a property of tending to accumulate in a site with abundant blood flow, such as a tumor, via a blood vessel. In the contrast-enhanced imaging in a case in which the iodine contrast agent is used, the first energy need only be set to be lower than the K absorption edge, and the second energy need only be set to be higher than the K absorption edge.

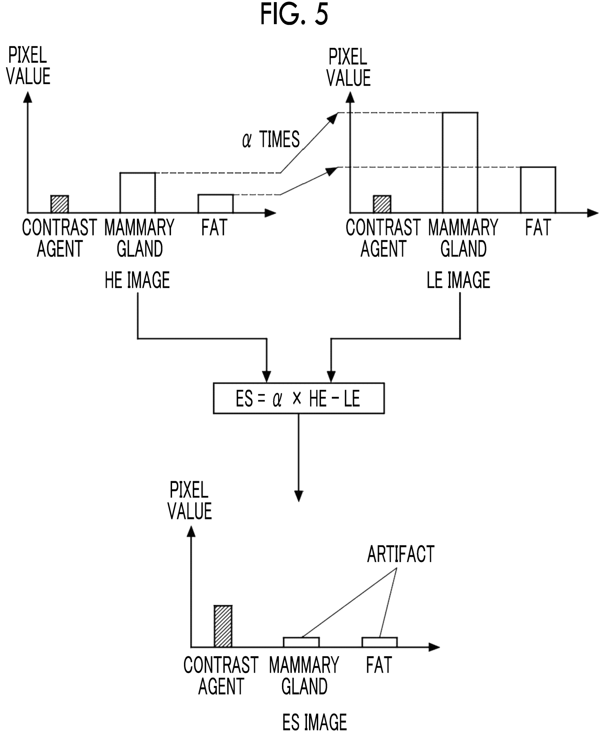

FIG. 5 conceptually describes the contrast enhancement by the energy difference processing. In the LE image, in addition to the mammary gland and fat, the absorption of the contrast agent is also emphasized. On the other hand, in the HE image, the radiation absorption of the mammary gland and fat is reduced, and the influence of the contrast agent is relatively small. This is because the mammary gland and fat exhibit similar radiation absorption characteristics in both the LE image and the HE image, and the components of the mammary gland and fat are suppressed by the difference processing between the LE image and the HE image.

On the other hand, since the contrast agent exhibits a significant difference in absorption characteristics at an energy near the K absorption edge, the site at which the contrast agent is present is emphasized by performing the difference processing between the LE image and the HE image. As a result, by the energy difference processing, the site at which the contrast agent is present is easily visible while suppressing the influence of the mammary gland or fat.

Specifically, an energy difference image (hereinafter, referred to as an "ES image") is generated by performing the difference processing between the LE image and the HE image based on Equation (1). $ES = α \times HE - LE$

Here, ES represents a pixel value of the ES image, LE represents a pixel value of the LE image, and HE represents a pixel value of the HE image. α is a coefficient. Equation (1) represents that the pixel value of the ES image is determined by subtracting the pixel value of the LE image from the value obtained by multiplying the pixel value of the HE image by the weight coefficient α for the corresponding pixel values of the LE image and the HE image. The weight coefficient α is not limited to the HE image, and may be multiplied by at least one of the HE image or the LE image.

The weight coefficient α is determined by Equation (2) using an absorption coefficient µ_{g}^{L} of the mammary gland for the radiation R having the first energy, an absorption coefficient µₐ^{L} of the fat for the radiation R having the first energy, an absorption coefficient µ_{g}^{H} of the mammary gland for the radiation R having the second energy, and an absorption coefficient µₐ^{H} of the fat for the radiation R having the second energy. $α = \left({{μ}_{g}}^{L}-{{μ}_{a}}^{L}\right) / \left({{μ}_{g}}^{H}-{{μ}_{a}}^{H}\right)$

In the energy difference processing, a problem occurs in that a low-frequency non-uniformity occurs in the energy difference image due to factors such as a change in thickness, scattered rays, and beam hardening, and this appears as an artifact in the ES image.

For example, in a case of the biopsy imaging, the problem of the artifact is significant due to the following factors. In the biopsy imaging, since the opening 30A is provided in the compression plate 30, a region in which the thickness is uneven occurs in a part of the breast M. In addition, in imaging from an oblique direction, the change in thickness of the breast M along the path of the radiation R increases. In a case in which the radiation R is obliquely incident, the path through which the radiation R passes is lengthened, and as a result, the pixel value of the radiation image is reduced. In addition, as the thickness of the breast M increases, the attenuation of the radiation R increases, and similarly, the pixel value is reduced. As described above, the change in the thickness of the breast M or the oblique incidence component of the radiation R changes the transmission path, which changes the pixel value and is one of the causes of the artifact.

In addition, in the biopsy imaging, a grid for removing scattered rays is not used in many cases, and the influence of the scattered rays is large. In the image of the breast M, the pixel value varies for each region due to the influence of the scattered rays, which is one of the causes of the artifact. For example, on an inner side of the breast M or the chest wall side, a large number of scattered rays are generated, and the pixel value is large. On the other hand, in the end part or the nipple side, the scattered rays easily escape outward, and the pixel value is reduced. As the thickness of the breast M increases, the number of scattered rays increases, and the artifact is significant.

Further, the influence of the beam hardening is also one of the causes of the occurrence of the artifact. The beam hardening is a phenomenon in which, in a case in which the radiation passes through the subject, the low-energy component is attenuated and the high-energy component is relatively increased, and as a result, the signal ratio between the LE image and the HE image is changed, and the weight coefficient α does not match the original appropriate value. As a result, the artifact is likely to appear. For example, due to the influence of the beam hardening, the absorption characteristics of the mammary gland or the fat are in a state different from the original energy characteristics, and thus the mammary gland component is not completely suppressed and remains as a factor even in a case in which the energy difference processing is performed.

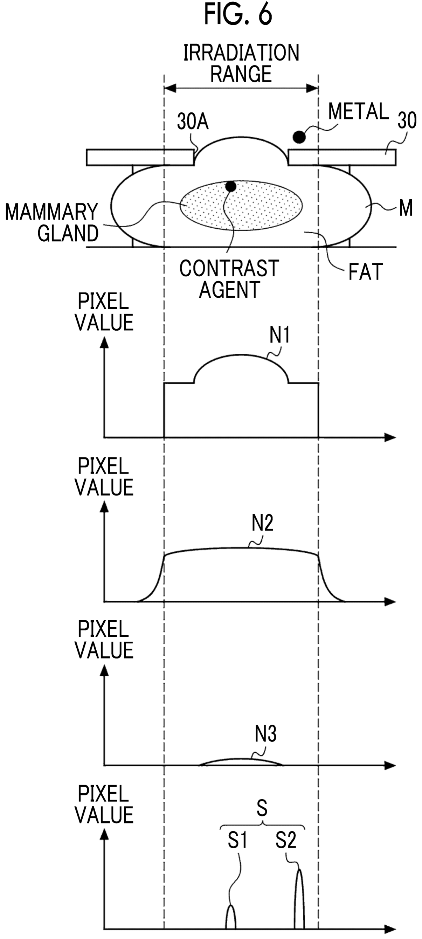

FIG. 6 shows an example of the artifact component and the signal component included in the ES image in a case of the biopsy imaging. In FIG. 6, N1 to N3 are artifact components, and S is a signal component. N1 is a part of a non-uniformity component due to the thickness of the breast M. N2 is a part of a non-uniformity component due to the scattered rays. N3 is a residual component of the mammary gland. The signal component S includes a signal component S1 due to the contrast agent and a signal component S2 due to a metal such as the biopsy needle 33.

In the present embodiment, the information processing apparatus 12 generates the ES image in which the artifact is removed with high accuracy by performing the image processing based on the LE image and the HE image.

The mammography apparatus 10 and the information processing apparatus 12 are connected by wired communication or wireless communication. The radiation image generated by the radiation detector 20 in the mammography apparatus 10 is output to the information processing apparatus 12 by wired communication or wireless communication via a communication interface (I/F) (not shown).

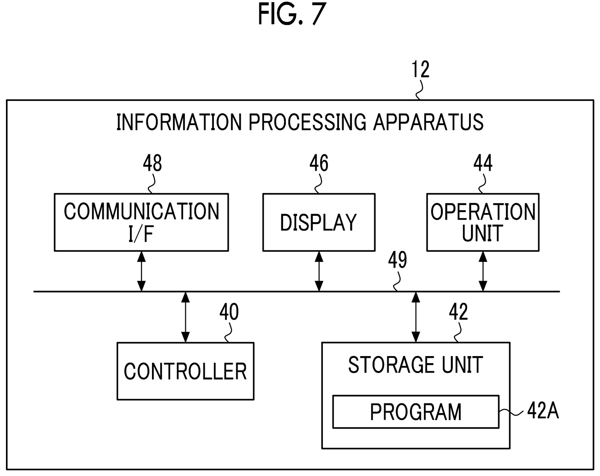

FIG. 7 shows an example of the configuration of the information processing apparatus 12. The information processing apparatus 12 comprises a controller 40, a storage unit 42, an operation unit 44, a display 46, and a communication I/F 48. The controller 40, the storage unit 42, the operation unit 44, the display 46, and the communication I/F 48 are connected to each other via a bus 49 such that various kinds of information can be exchanged.

The controller 40 controls an overall operation of the radiation image capturing system 2. The controller 40 is configured as, for example, a computer comprising a central processing unit (CPU), a read only memory (ROM), and a random access memory (RAM). The controller 40 is an example of a "processor" according to the technology of the present disclosure.

The storage unit 42 stores information related to radiography, the radiation image acquired from the mammography apparatus 10, and the like. The storage unit 42 stores a program 42A for the controller 40 to perform various types of information processing described below. The storage unit 42 is, for example, a nonvolatile storage device such as a hard disk drive (HDD) or a solid state drive (SSD). The storage unit 42 is an example of a "memory" according to the technology of the present disclosure.

The operation unit 44 includes input devices such as various buttons, switches, a touch panel, a touch pen, and a mouse, which are operated by the user. The display 46 displays information related to the imaging, the radiation image obtained by the imaging, and the like.

The communication I/F 48 performs communication of various kinds of data, such as information related to the radiography and the radiation image, with the mammography apparatus 10, the RIS, the PACS, and the like via wired communication or wireless communication.

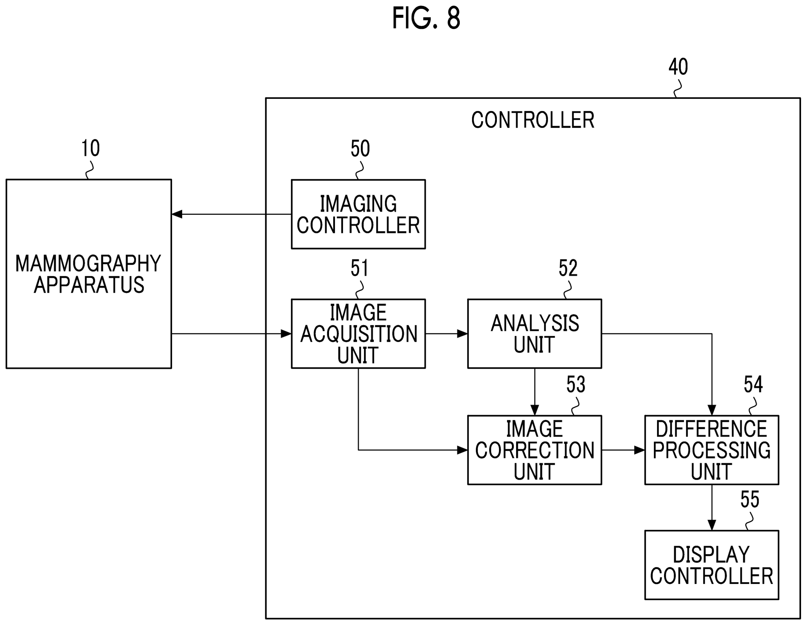

FIG. 8 shows an example of functions implemented by the controller 40 of the information processing apparatus 12. The controller 40 is configured to realize various functions by executing the processing based on the program 42A stored in the storage unit 42. The controller 40 functions as an imaging controller 50, an image acquisition unit 51, an analysis unit 52, an image correction unit 53, a difference processing unit 54, and a display controller 55.

The imaging controller 50 controls the mammography apparatus 10 to perform radiography in response to an operation of the operation unit 44 by a user such as a radiologic technologist. Specifically, the imaging controller 50 causes the mammography apparatus 10 to perform the radiography using the radiation R having the first energy and the radiography using the radiation R having the second energy.

Before the imaging using the mammography apparatus 10 is started, the user injects the contrast agent into one of the left and right breasts M of the person under the examination, positions the breast M into which the contrast agent has been injected on the imaging table 24, and compresses the breast M with the compression plate 30.

In a case in which the imaging instruction is received from the operation unit 44, the imaging controller 50 transmits the imaging instruction signal to the mammography apparatus 10. The mammography apparatus 10 performs the imaging of the LE image by irradiating the breast M with the radiation R having the first energy, and then performs the imaging of the HE image by irradiating the breast M with the radiation R having the second energy. The HE image may be imaged before the LE image.

The image acquisition unit 51 acquires the LE image and the HE image obtained as a result of the radiography using the mammography apparatus 10, and supplies the acquired LE image and HE image to the analysis unit 52 and the image correction unit 53.

The analysis unit 52 generates an initial ES image by performing the above-described energy difference processing using the LE image and the HE image, and extracts the artifact component based on the generated initial ES image. Specifically, the analysis unit 52 extracts the above-described non-uniformity components N1 and N2 based on the initial ES image.

The image correction unit 53 corrects the LE image and the HE image based on the artifact component extracted by the analysis unit 52. Specifically, the image correction unit 53 corrects the LE image and the HE image to remove the non-uniformity components N1 and N2. The image correction unit 53 supplies a corrected LEc image obtained by correcting the LE image and a corrected HEc image obtained by correcting the HE image to the difference processing unit 54.

The difference processing unit 54 generates the ES image in which the artifact is removed by performing the above-described energy difference processing based on the LEc image and the HEc image. The display controller 55 performs display control of displaying the ES image on the display 46.

In the present embodiment, the analysis unit 52 calculates a correction coefficient k for suppressing the residual component N3 of the mammary gland based on the non-uniformity component N1. The correction coefficient k is a coefficient for correcting the weight coefficient α used in the energy difference processing. The difference processing unit 54 performs the energy difference processing after correcting the weight coefficient α using the correction coefficient k.

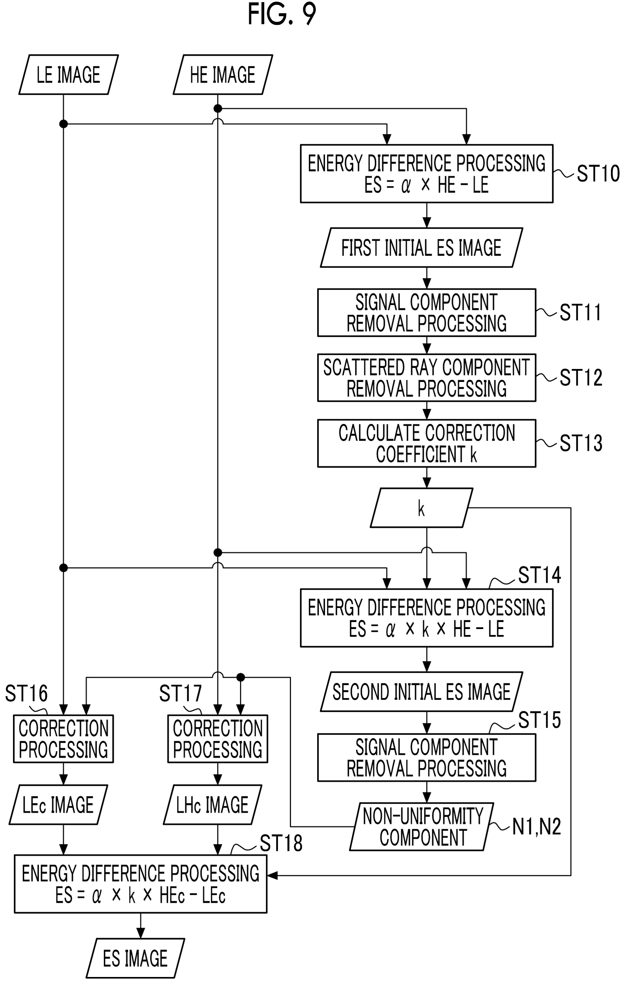

FIG. 9 describes in detail the processing of generating the ES image by the analysis unit 52, the image correction unit 53, and the difference processing unit 54. First, the analysis unit 52 generates a first initial energy difference image (hereinafter, referred to as a "first initial ES image") by performing the energy difference processing based on Equation (1) using the LE image and the HE image supplied from the image acquisition unit 51 (step ST10). The first initial ES image includes the above-described artifact components N1 to N3 and the signal component S.

Next, the analysis unit 52 performs the signal component removal processing of removing the signal component S from the first initial ES image (step ST11). Since the signal component S is a high-frequency component, for example, the signal component S is removed using a high-frequency cut filter. As a result, the first initial ES image is an image in which the signal component S is removed and the artifact components N1 to N3 remain.

Next, the analysis unit 52 performs the scattered ray component removal processing of removing the scattered ray component N2 from the first initial ES image (step ST12). Since the scattered ray component N2 is a low-frequency component, for example, the scattered ray component N2 is removed using a predetermined low-frequency cut filter. As a result, the first initial ES image is an image in which the signal component S and the scattered ray component N2 are removed and the artifact components N1 and N3 remain. The analysis unit 52 may use a low-frequency cut filter that takes into consideration the thickness of the breast M. This is because the scattered component increases as the thickness of the breast M increases. Since the thickness of the breast M corresponds to a distance between the surface of the imaging table 24 and the compression plate 30, the cutoff frequency of the low-frequency cut filter may be set based on this distance.

Next, the analysis unit 52 calculates the above-described correction coefficient k based on the thickness component of the breast M included in the first initial ES image (step ST13). The first initial ES image in which the signal component removal processing and the scattered ray component removal processing are performed includes the artifact components N1 and N3, but the residual component N3 of the mammary gland is smaller than the non-uniformity component N1 due to the thickness of the breast M. Therefore, the analysis unit 52 determines the correction coefficient k such that the artifact components N1 and N3 are regarded as the thickness components, and the residual component N3 of the mammary gland generated by the change in thickness is removed by the energy difference processing.

Next, the analysis unit 52 generates a second initial energy difference image (hereinafter, referred to as a "second initial ES image") by performing the energy difference processing again using the LE image and the HE image after correcting the weight coefficient α using the correction coefficient k (step ST14). Specifically, the analysis unit 52 performs the difference processing between the LE image and the HE image based on Equation (3). $ES = α \times k \times HE - LE$

As a result, the second initial ES image is an image in which the residual component N3 of the mammary gland is removed and the signal component S and the artifact components N1 and N2 (that is, the non-uniformity components N1 and N2) remain.

Next, the analysis unit 52 performs the signal component removal processing of removing the signal component S from the second initial ES image (step ST15). The signal component removal processing is the same as the signal component removal processing on the first initial ES image (step ST11). As a result, the non-uniformity components N1 and N2 are extracted from the second initial ES image.

Next, the image correction unit 53 performs the correction processing of removing the non-uniformity components N1 and N2 from the LE image (step ST16), and performs the correction processing of removing the non-uniformity components N1 and N2 from the HE image (step ST17). For example, the image correction unit 53 removes the non-uniformity components N1 and N2 by adding a correction image in which the signs of the non-uniformity components N1 and N2 are inverted to each of the LE image and the HE image.

Next, the difference processing unit 54 performs the energy difference processing using the LEc image and the HEc image after correcting the weight coefficient α using the correction coefficient k, to generate the ES image (step ST18). Specifically, the difference processing unit 54 performs the difference processing between the LEc image and the HEc image based on Equation (4). $ES = α \times k \times HEc - LEc$

As described above, the ES image in which the non-uniformity components N1 and N2 and the residual component N3 of the mammary gland (that is, the artifact components N1 to N3) are removed is generated.

As described above, in the present embodiment, the initial ES image is generated by performing the difference processing between the LE image and the HE image, the artifact component is extracted from the initial ES image, and the ES image is generated by performing the difference processing again based on the artifact component, so that the artifact can be highly accurately removed from the ES image. The ES image refers to both the first initial ES image and the second initial ES image. In addition, performing the difference processing again based on the artifact component includes at least one of performing the difference processing after correcting the LE image and the HE image or correcting the weight coefficient α to perform the difference processing.

### [Modification Example]

In the above-described embodiment, the difference processing unit 54 performs the energy difference processing after correcting the weight coefficient α using the correction coefficient k, but the difference processing unit 54 may generate the ES image by performing the energy difference processing without correcting the weight coefficient α. This is because the residual component N3 of the mammary gland has a lower signal intensity than the non-uniformity components N1 and N2.

In addition, in the above-described embodiment, the first initial ES image and the second initial ES image are generated, but only the first initial ES image may be generated as the initial ES image, and the LE image and the HE image may be corrected based on the artifact components N1 to N3 remaining after the signal component S is removed from the first initial ES image. This is because the residual component N3 of the mammary gland has a lower signal intensity than the non-uniformity components N1 and N2, and the artifact components N1 to N3 can be regarded as the non-uniformity components. In addition, the residual component N3 of the mammary gland may be suppressed by the signal component removal processing on the first initial ES image together with the signal component S.

In addition, in the above-described embodiment, the signal component removal processing and the scattered ray component removal processing are performed using the frequency cut filter, but instead of this, signal processing using a machine-learned model or the like, signal processing using pattern recognition, or the like may be used.

In the above-described embodiment, each processing executed by the controller 40 is executed by any computer. Any computer may execute these processes by a processor as hardware, a program as software, or a combination thereof. In such a case, the processor is configured to execute various processes in the above-described embodiment in cooperation with the program, and may function as each unit or each means in the above-described embodiment. In addition, the execution order of the processes by the processor is not limited to the above order, and may be changed as appropriate. Any computer may be a general-purpose computer, a dedicated computer, a workstation, or another system that can execute each process.

The processor may be configured by one or more kinds of hardware, and the type of hardware is not limited. For example, the processor may be configured by hardware such as a CPU, a micro processing unit (MPU), a programmable logic device including a field programmable gate array (FPGA), a dedicated circuit for executing specific processing including an application specific integrated circuit (ASIC), a graphic processing unit (GPU), a neural processing unit (NPU), or the like. Further, the type of hardware may be a combination of different types of hardware. In a case in which the plurality of types of hardware are configured to execute one or a plurality of processes of a certain processor, the plurality of types of hardware may be present in devices physically separated from each other or may be present in the same device. Further, in any of the embodiments, the order of each processing performed by the processor is not limited to the above-described order, and may be changed as appropriate. The hardware is configured by an electric circuit (circuitry) in which circuit elements, such as semiconductor elements, are combined, or the like.

Furthermore, the program may be software such as firmware or microcode. The program may be, for example, a group of program modules, and each function thereof may be implemented by a processor configured to execute each function. The program may be program code or a plurality of code segments stored in one or more non-transitory computer-readable media (for example, a storage medium or other storage). The program may be stored in the plurality of non-transitory computer-readable media present in devices physically separated from each other. The program code or the code segment may represent any combination of procedures, functions, subprograms, routines, subroutines, modules, software packages, classes, instructions, data structures, or program statements. The program code or the code segment may be connected to another code segment or a hardware circuit by transmitting and receiving information, data, arguments, parameters, or contents in the memory.

Further, in the above-described embodiments, the aspect has been described in which the program 42A is stored in the storage unit 42 in advance. However, the present disclosure is not limited thereto. The program 42A may be provided in a form of being recorded on a recording medium, such as a compact disc read only memory (CD-ROM), a digital versatile disc read only memory (DVD-ROM), and a universal serial bus (USB) memory. In addition, the program 42A may be downloaded from an external device via a network.

The technology of the present disclosure extends to any program product. The program product includes products in any aspect for providing the program. For example, the program product includes a program provided through a network such as the Internet, a non-transitory computer-readable recording medium, such as a CD-ROM, a DVD, and a USB memory in which the program is stored, and the like.

The following technology can be understood based on the above description.

### [Supplementary Note 1]

An information processing apparatus comprising a processor, in which the processor is configured to acquire a low-energy image captured by irradiating a subject into which a contrast agent has been injected with radiation having a first energy, and a high-energy image captured by irradiating the subject with radiation having a second energy higher than the first energy, generate an initial energy difference image by performing difference processing on the low-energy image and the high-energy image, extract an artifact component from the initial energy difference image, and generate an energy difference image by performing the difference processing again based on the artifact component.

### [Supplementary Note 2]

The information processing apparatus according to Supplementary Note 1, in which the processor is configured to generate the energy difference image by correcting the low-energy image and the high-energy image based on the artifact component and then performing the difference processing.

### [Supplementary Note 3]

The information processing apparatus according to Supplementary Note 1 or 2, in which the processor is configured to extract, as the artifact component, a remaining component obtained by performing, on the initial energy difference image, signal component removal processing of removing a signal component corresponding to the contrast agent.

### [Supplementary Note 4]

The information processing apparatus according to Supplementary Note 3, in which the processor is configured to perform the signal component removal processing using a high-frequency cut filter.

### [Supplementary Note 5]

The information processing apparatus according to Supplementary Note 1, in which the initial energy difference image includes a first initial energy difference image and a second initial energy difference image, and the processor is configured to generate the first initial energy difference image by multiplying at least one of the low-energy image or the high-energy image by a weight coefficient and performing the difference processing, and generate the second initial energy difference image by performing the difference processing again using the weight coefficient corrected based on a remaining component obtained by performing, on the first initial energy difference image, signal component removal processing of removing a signal component corresponding to the contrast agent and scattered ray component removal processing of removing a scattered ray component.

### [Supplementary Note 6]

The information processing apparatus according to Supplementary Note 5, in which the processor is configured to extract, as the artifact component, a remaining component obtained by performing the signal component removal processing on the second initial energy difference image.

### [Supplementary Note 7]

The information processing apparatus according to Supplementary Note 6, in which the processor is configured to generate the energy difference image by correcting the low-energy image and the high-energy image based on the artifact component and then performing the difference processing.

### [Supplementary Note 8]

The information processing apparatus according to any one of Supplementary Notes 5 to 7, in which the processor is configured to generate the energy difference image by performing the difference processing using the corrected weight coefficient.

### [Supplementary Note 9]

The information processing apparatus according to any one of Supplementary Notes 5 to 8, in which the processor is configured to correct the weight coefficient based on a thickness component of the subject included in the first initial energy difference image.

### [Supplementary Note 10]

The information processing apparatus according to Supplementary Note 9, in which the thickness component is a remaining component obtained by performing the signal component removal processing and the scattered ray component removal processing on the first initial energy difference image.

### [Supplementary Note 11]

The information processing apparatus according to any one of Supplementary Notes 5 to 10, in which the processor is configured to perform the signal component removal processing using a high-frequency cut filter.

### [Supplementary Note 12]

The information processing apparatus according to any one of Supplementary Notes 1 to 11, in which the subject is a breast.

### Explanation of References

2: radiation image capturing system
10: mammography apparatus
12: information processing apparatus
20: radiation detector
20A: detection surface
24: imaging table
26: base
27: shaft portion
28: arm portion
29: radiation source
30: compression plate
30A: opening
32: compression unit
33: biopsy needle
40: controller
42: storage unit
42A: program
44: operation unit
46: display
48: communication I/F
49: bus
50: imaging controller
51: image acquisition unit
52: analysis unit
53: image correction unit
54: difference processing unit
55: display controller
M: breast
R: radiation
S: signal component
N1 to N3: artifact component

## Claims

1. An information processing apparatus (12) comprising:
a processor (40),
wherein the processor is configured to:
acquire a low-energy image captured by irradiating a subject (M) into which a contrast agent has been injected with radiation having a first energy, and a high-energy image captured by irradiating the subject with radiation having a second energy higher than the first energy;
generate an initial energy difference image by performing difference processing on the low-energy image and the high-energy image;
extract an artifact component (N1-N3) from the initial energy difference image; and
generate an energy difference image by performing the difference processing again based on the artifact component.

2. The information processing apparatus according to claim 1,
wherein the processor (40) is configured to generate the energy difference image by correcting the low-energy image and the high-energy image based on the artifact component (N1-N3) and then performing the difference processing.

3. The information processing apparatus according to claim 1,
wherein the processor (40) is configured to extract, as the artifact component, a remaining component obtained by performing, on the initial energy difference image, signal component removal processing of removing a signal component corresponding to the contrast agent.

4. The information processing apparatus according to claim 3,
wherein the processor (40) is configured to perform the signal component removal processing using a high-frequency cut filter.

5. The information processing apparatus according to claim 1,
wherein the initial energy difference image includes a first initial energy difference image and a second initial energy difference image, and
the processor is configured to:
generate the first initial energy difference image by multiplying at least one of the low-energy image or the high-energy image by a weight coefficient and performing the difference processing; and
generate the second initial energy difference image by performing the difference processing again using the weight coefficient corrected based on a remaining component obtained by performing, on the first initial energy difference image, signal component removal processing of removing a signal component corresponding to the contrast agent and scattered ray component removal processing of removing a scattered ray component.

6. The information processing apparatus according to claim 5,
wherein the processor (40) is configured to extract, as the artifact component, a remaining component obtained by performing the signal component removal processing on the second initial energy difference image.

7. The information processing apparatus according to claim 6,
wherein the processor (40) is configured to generate the energy difference image by correcting the low-energy image and the high-energy image based on the artifact component and then performing the difference processing.

8. The information processing apparatus according to claim 7,
wherein the processor (40) is configured to generate the energy difference image by performing the difference processing using the corrected weight coefficient.

9. The information processing apparatus according to claim 5,
wherein the processor (40) is configured to correct the weight coefficient based on a thickness component of the subject included in the first initial energy difference image.

10. The information processing apparatus according to claim 9,
wherein the thickness component is a remaining component obtained by performing the signal component removal processing and the scattered ray component removal processing on the first initial energy difference image.

11. The information processing apparatus according to any one of claims 5 to 10,
wherein the processor (40) is configured to perform the signal component removal processing using a high-frequency cut filter.

12. The information processing apparatus according to claim 1,
wherein the subject is a breast (M).

13. An information processing method comprising:
acquiring a low-energy image captured by irradiating a subject (M) into which a contrast agent has been injected with radiation having a first energy, and a high-energy image captured by irradiating the subject with radiation having a second energy higher than the first energy;
generating an initial energy difference image by performing difference processing on the low-energy image and the high-energy image;
extracting an artifact component (N1-N3) from the initial energy difference image; and
generating an energy difference image by performing the difference processing again based on the artifact component.

14. A program causing a computer to execute a process comprising:
acquiring a low-energy image captured by irradiating a subject (M) into which a contrast agent has been injected with radiation having a first energy, and a high-energy image captured by irradiating the subject with radiation having a second energy higher than the first energy;
generating an initial energy difference image by performing difference processing on the low-energy image and the high-energy image;
extracting an artifact component (N1-N3) from the initial energy difference image; and
generating an energy difference image by performing the difference processing again based on the artifact component.
